# EUROPÄISCHE PATENTANMELDUNG

(11) **EP 1 760 469 A1**
(43) Veröffentlichungstag der Anmeldung: **07.03.2007**
(21) Anmeldenummer: 05018973.7
(22) Anmeldetag: 01.09.2005
(51) Int. Cl.: G01N 35/00, G01N 21/86, A61B 5/00

(54) **Testsystem zur Untersuchung von Körperflüssigkeiten mit einem drehbaren Optikelement**

(71) Anmelder: F.HOFFMANN-LA ROCHE AG, 4070 Basel (CH); Roche Diagnostics GmbH, 68305 Mannheim (DE)
(72) Erfinder: Petrich, Wolfgang, Priv-Doz. Dr., 76669 Bad Schönborn (DE)
(74) Vertreter: Pfiz, Thomas

(57) **Zusammenfassung**

Die Erfindung betrifft ein Testsystem zur Untersuchung von Körperflüssigkeiten, insbesondere Blut, mit einem eine Vielzahl von Testelementen (20) tragenden Testelementband (18), einer Bandumlenkung (22) für das Testelementband (18) zur gezielten Applikation von Körperflüssigkeit und einer Lichtquelle (32) sowie einem Detektor (40) zur optischen Untersuchung von mit Körperflüssigkeit beaufschlagten Testelementen (20). Erfindungsgemäß wird vorgeschlagen, dass die Bandumlenkung (22) ein zum Transport des Testelementbands (18) drehbares Optikelement (24) als Umlenkrolle im Strahlengang zwischen Lichtquelle (32) und Detektor (40) aufweist.

## Beschreibung

Die Erfindung betrifft ein Testsystem zur Untersuchung von Körperflüssigkeiten, insbesondere Blut, mit einem eine Vielzahl von Testelementen tragenden, vorzugsweise in einer Bandkassette aufgewickelten Testelementband, einer Bandumlenkung für das Testelementband zur gezielten Applikation von Körperflüssigkeit und einer Lichtquelle sowie einem Detektor zur optischen Untersuchung von mit Körperflüssigkeit beaufschlagten Testelementen.

Zur Blutzucker-Selbstkontrolle von Diabetikern sind gleichsam als Minilabore arbeitende Handgeräte bekannt, mit denen auch von Laien die erforderlichen Schritte einfach und schnell vorgenommen werden können. Um die herkömmlichen Teststreifen zu ersetzen, wird in der WO 2004/047642 vorgeschlagen, anstelle einzelner Teststreifen ein aufgewickeltes Testband zu verwenden, auf dem eine Vielzahl von mit einer geeigneten Testchemie versehenen Testfeldern fortlaufend angeordnet sind. Die Körperflüssigkeit wird auf einem durch Bandvorlauf über eine Spitze in eine aktive Position gebrachten Testfeld appliziert und analysiert. Einzelheiten zur Blutgewinnung sowie zu bekannten Testmedien und Nachweissystemen insbesondere für Blutglucose sind dort angegeben, worauf hier Bezug genommen und der diesbezügliche Inhalt in diese Anmeldung aufgenommen wird. Aus dieser Druckschrift geht auch hervor, dass eine transparente Spitze eingesetzt werden kann, um die Geräteoptik direkt anzukoppeln. Als Randbedingungen sind hierbei zu beachten, dass die Bauhöhe der Umlenkspitze und deren Öffnungswinkel möglichst klein sein sollte, zugleich aber eine Möglichkeit bestehen sollte, das Testfeld optisch zu untersuchen, beispielsweise auf Basis einer Remissionsmessung oder einer Fluoreszenzmessung.

Ausgehend hiervon liegt der Erfindung die Aufgabe zugrunde, die genannten-Nachteile zu vermeiden und ein System der eingangs genannten Art zu verbessern und zu vereinfachen, insbesondere auch Beanspruchungen des Testbands zu reduzieren, so dass ein zuverlässiger Messablauf in einem kompakten Gerät erreichbar ist.

Zur Lösung dieser Aufgabe wird die im Patentanspruch 1 bzw. 10 angegebene Merkmalskombination vorgeschlagen. Vorteilhafte Ausgestaltungen und Weiterbildungen der Erfindung ergeben sich aus den abhängigen Ansprüchen.

Der Kern der Erfindung besteht darin, eine sowohl zum Bandtransport als auch zur optischen Untersuchung vorteilhafte Umlenkung für das Testband zu schaffen. Dementsprechend wird erfindungsgemäß vorgeschlagen, dass die Bandumlenkung ein zum Transport des Testelementbands drehbares Optikelement im Strahlengang zwischen Lichtquelle und Detektor aufweist. Durch das beim Bandtransport mitrotierende Optikelement ist es möglich, Reibungsverluste bei der Bandumlenkung deutlich zu verringern, so dass die Gefahr einer Bandverformung bzw. eines Bandrisses erheblich verringert wird. Damit lassen sich auch dünnere Trägerbänder einsetzen, um die Kapazität bei gegebenem Bauvolumen steigern zu können. Zudem kann der Bandtransport mit einer geringeren Motorleistung bewerkstelligt werden, bei gleichzeitig geringerem Stromverbrauch. Durch das transparente Optikelement wird auch ein einfacher optischer Zugang zu dem Testelement geschaffen, ohne dass dieses zu einer entfernten Messposition weitertransportiert werden müsste.

Vorteilhafterweise besitzt das lichtdurchlässige Optikelement eine an das Testelementband anliegende und in dessen Transportrichtung drehbare Umlenkfläche, so dass eine direkte optische Ankopplung in der Messposition und eine mitrotierende Umlenkung beim Bandvorlauf gewährleistet ist.

Eine weitere vorteilhafte Ausführung sieht vor, dass das Optikelement durch eine das Licht der Lichtquelle auf das jeweils zu untersuchende Testelement des Testelementbands bündelnde Linse gebildet ist. Damit lassen sich Abbildungseffekte für eine Miniaturisierung Messfeldes ausnutzen.

Eine weitere Verbesserung in baulicher Hinsicht wird dadurch erreicht, dass das Optikelement als eine um ihre Längsachse frei drehbar gelagerte, das Testelementband mantelseitig führende zylinderförmige Linse ausgebildet ist.

Für den Bandtransport ist es günstig, wenn das Optikelement einen vorzugsweise kreisförmigen, elliptischen oder vieleckigen drehsymmetrischen Querschnitt besitzt.

Für eine einfache und ausreichend reibungsarme Lagerung kann das Optikelement in einem Gleitlager, vorzugsweise einem Zapfenlager oder Spitzenlager drehbar gelagert sein.

In einer weiteren vorteilhaften Ausgestaltung der optischen Ankopplung ist es vorgesehen, dass das Optikelement in Kombination mit einer der Lichtquelle nachgeordneten und/oder dem Detektor vorgeordneten Linse eine Abbildungsoptik bildet.

Zur Abschirmung von Umgebungslicht ist es von Vorteil, wenn eine Blende im Bereich einer Zwischenabbildung zwischen Testelementband und Detektor angeordnet ist.

Gemäß einem weiteren Aspekt der Erfindung ist der Detektor außerhalb des Ausfallsbereichs bzw. der optischen Achse des von dem Testelement spiegelnd reflektierten Lichts bzw. Lichtbündels der Lichtquelle angeordnet. Auf diese Weise kann eine rein geometrische Unterscheidung des diffus (in alle Richtungen) reflektierten Lichts erreicht werden, welches die eigentliche Information aus der Reagenzschicht des Testfelds über den Analyten liefert, während die spiegelnde Reflektion zumindest im Wesentlichen nicht in den Eintrittsquerschnitt des Detektors gelangt.

Eine solche geometrische Trennung lässt sich dadurch realisieren, dass die Lichtquelle unter einem Einfallswinkel und der Detektor unter einem im Vergleich dazu größeren Empfangswinkel in einer gemeinsamen Ebene einseitig von dem Einfallslot auf das Testelementband ausgerichtet sind.

Eine weitere vorteilhafte Anordnung sieht vor, dass die Lichtquelle und der Detektor in einem gemeinsamen Halbraum im seitlichen Abstand von einer durch die Mittellinie des Testelementbands im Bereich der Bandumlenkung aufgespannten Begrenzungsebene angeordnet sind. Das regulär reflektierte Licht wird dabei im Wesentlichen in den anderen Halbraum abgestrahlt, ohne in den Detektor zu gelangen.

In baulicher Hinsicht ist es von besonderem Vorteil, wenn der Lichtempfänger und der Detektor auf einer Träger in einem die Bandkassette aufnehmenden Gerät angeordnet und über eine in der Bandkassette integrierte Umlenkoptik, insbesondere einen Spiegel auf die Bandumlenkung ausgerichtet sind.

Im Folgenden wird die Erfindung anhand der in der Zeichnung in schematischer Weise dargestellten Ausführungsbeispiele näher erläutert. Es zeigen
- Fig. 1: ein Blutzuckermessgerät mit einer Bandumlenkung zur Anwendung eines Testelementbands in perspektivischer Ansicht;
- Fig. 2: die als drehbare Zylinderlinse ausgebildete Bandumlenkung in Verbindung mit einer reflektometrischen Messeinrichtung in einem vereinfachten Querschnitt;
- Fig. 3: einen Längsschnitt der Anordnung nach Fig. 2;
- Fig. 4: eine weitere Ausführungsform einer optischen Umlenkrolle in einer Fig. 3 entsprechenden Darstellung;
- Fig. 5: eine mehreckige Umlenkrolle im Profil;
- Fig. 6: ein die Umlenkrolle enthaltendes Kassettenteil für das Testelementband in einer perspektivischen Ansicht;

- Fig. 7: eine Messanordnung des Blutzuckermessgeräts in einer ausschnittsweisen perspektivischen Darstellung; und
- Fig. 8: eine teilweise geschnittene Seitenansicht der Anordnung nach Fig. 7.

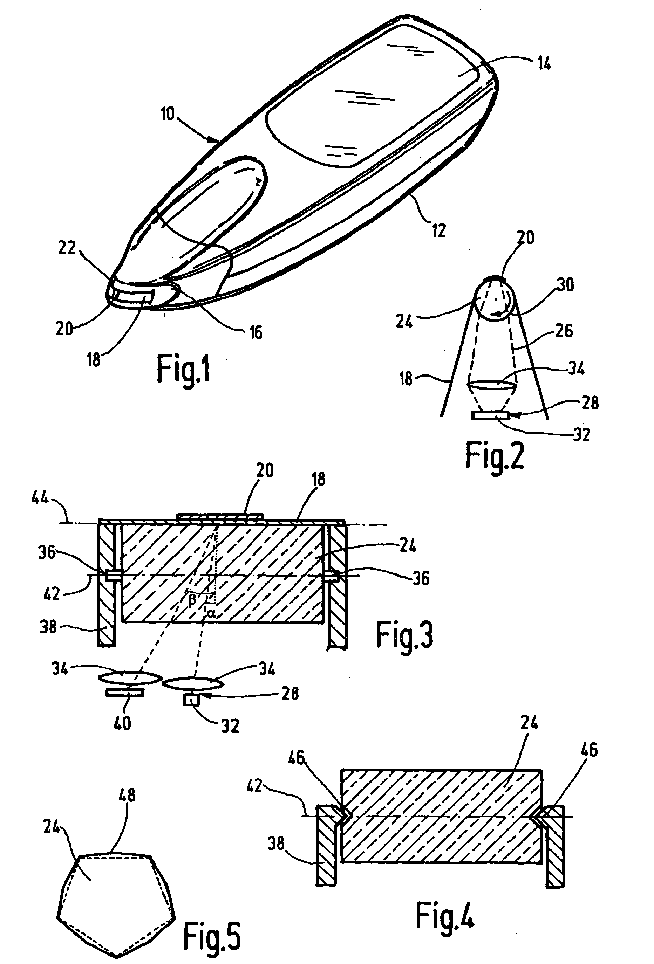

Fig. 1 veranschaulicht ein Testsystem für Körperflüssigkeiten, speziell ein Blutzuckermessgerät 10 in Form eines tragbaren Handgeräts. Dieses umfasst ein Gerätegehäuse 12 mit Anzeige 14, in welchem eine Bandkassette 16 als Verbrauchsteil einsetzbar ist. Die Kassette enthält ein Testelementband 18, welches zweckmäßig abschnittsweise mit Testelementen bzw. Testfeldern 20 versehen ist. Die Testelemente 20 lassen sich mit Körperflüssigkeit (Blut) beaufschlagen, um einen darin enthaltenen Analyten (Glucose) über eine reflektometrische Messung quantitativ zu erfassen.

Die einzelnen Testelemente 20 werden durch Vorspulen des Testbandes 18 über eine der Grundform nach V-förmige Bandumlenkung 22 sukzessive in eine Aufnahmeposition im Umlenkbereich gebracht, in der sie für eine gezielte Applikation einer kleinen Menge von Körperflüssigkeit vorderseitig zugänglich sind. Die optische Vermessung erfolgt von der Bandrückseite her durch die Bandumlenkung 22 hindurch, wobei das Testelementband 18 eine transparente Trägerfolie oder einen Bandausschnitt im Bereich der Testelemente 20 aufweisen kann. Nach der Messung wird das verbrauchte Nach der Messung wird das verbrauchte Testelement 20 auf eine Aufwickelspule der Bandkassette 16 aufgespult, während ein unverbrauchtes Testfeld auf dem anschließenden Bandabschnitt von einer Vorratsspule abgezogen wird. Auf diese Weise kann der Benutzer eine Vielzahl von Tests in einem automatischen Messablauf durchführen, ohne dass aufwändige Handhabungsschritte erforderlich wären.

Wie in Fig. 2 veranschaulicht, weist die Bandumlenkung 22 ein transparentes drehbares Linsenelement 24 im Strahlengang 26 der optischen Messeinrichtung 28 auf. Das kreiszylindrisch ausgebildete, aus Glas oder transparentem Kunststoff bestehende Linsenelement 24 bildet mit seinem Mantel 30 eine mit dem Testelementband 18 beim Bandtransport mitdrehende Umlenkfläche, so dass nur geringe Reibungsverluste auftreten. Zugleich bündelt das Linsenelement 24 das von der Lichtquelle 32 der Messeinrichtung 28 erzeugte Messlicht auf das jeweils in der Aufnahmeposition befindliche Testelement 20. Um die Lichtausbeute zu verbessern, kann zusätzlich eine Sammellinse 34 vorgeordnet sein.

Fig. 3 zeigt eine mögliche Gleitlagerung der zylinderförmigen Linse 24 in Form von stirnseitigen Zapfenlagern 36 in den Seitenwandungen 38 der Kassette 16. Weiterhin veranschaulicht Fig. 3 eine mögliche Anordnung von Lichtquelle 32 und Detektor 40 der Messeinrichtung 28 zur bevorzugten Erfassung des an dem Testelement 20 diffus reflektierten Messlichts. Zu diesem Zweck ist der Detektor 40 außerhalb der optischen Achse des von der zugewandten Rückseite des Testelements 20 spiegelnd reflektierten Lichts der Lichtquelle 32 angeordnet. Im gezeigten Fall sind die Lichtquelle 32 und der Detektor 40 mittig in einer Ebene ausgerichtet, die durch die Drehachse 42 der zylindrischen Linse 24 und die Scheitellinie 44 der Bandumlenkung aufgespannt wird, wobei der Detektor 40 unter einem im Vergleich zum Einstrahlwinkel α der Lichtquelle 32 größeren Empfangswinkel β angeordnet ist.

Fig. 4 zeigt eine weitere Alternative zur einfachen Drehlagerung der zylinderförmigen Linse 24. Bei diesem Beispiel sind stirnseitige Spitzenlager 46 für eine reibungsarme punktförmige Berührung vorgesehen. Wichtig für die Auslegung ist eine geringe Positionstoleranz, insbesondere senkrecht zur Drehachse 42, eine geringe Reibung sowie günstige Herstellbarkeit.

Wie in Fig. 5 gezeigt, kann die Linse bzw. Umlenkrolle 24 anstelle eines kreisförmigen Querschnitts auch eine mehreckige Geometrie besitzen. In der gezeigten Ausführung ist der Grundform nach ein Fünfeck vorgesehen, um so noch besser definierte Transportwege beim Bandtransport zu bewerkstelligen. Dabei sind die Seitenflächen 48 gerundet bzw. flach gewinkelt, um die optischen Pfade vorteilhaft zu gestalten.

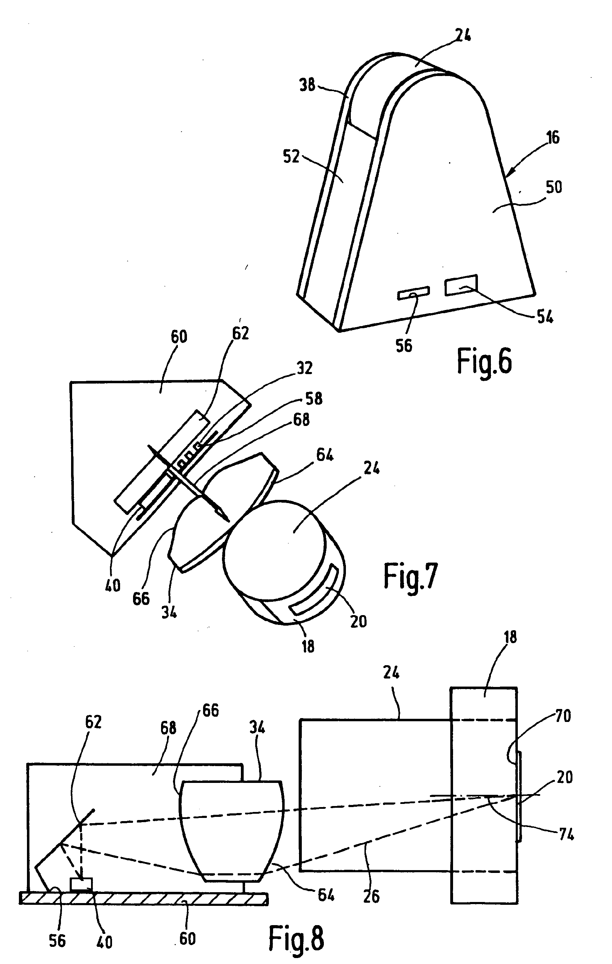

Gemäß Fig. 6 kann die Bandkassette 16 eine Kassettenspitze 50 mit V-förmig aufeinander zulaufenden Bandführungsflächen 52 aufweisen, wobei die zylinderförmige Linse 24 im Scheitelbereich sitzt. Innerhalb der Kassettenspitze 50 können nicht gezeigte Formschluss-Strukturen zur exakten Ausrichtung beim Andocken der Messeinrichtung 28 vorgesehen sein, wobei Wanddurchbrüche 54, 56 den Eingriff von Lichtquelle und Detektor von unten her erlauben.

Wie aus Fig. 7 und 8 ersichtlich, sind die Lichtquelle 32 - in Form von drei LEDs 58 - und der Detektor 40 auf einer geräteseitigen Hauptplatine 60 angeordnet, während der optische Lichtweg durch kassettenseitige Elemente bestimmt wird. Im Einzelnen sind dies ein Umlenkspiegel 62, eine Sammellinse 34 und die zylinderförmige Linse 24 als Umlenkrolle für das Testband 18. Die vorgeordnete Linse 34 weist dabei eine der Umlenkrolle zugewandte einfach konvexe Linsenfläche 64 und eine der Messeinrichtung 28 zugewandte doppelt konvexe Linsenfläche 66 auf, wobei eine Zentralblende 68 zwischen den Linsensegmenten die Lichtquelle 32 von dem Detektor 40 trennt.

Auch hier ist durch die geometrische Anordnung der optischen Elemente sichergestellt, dass nur diffus reflektiertes Licht von der Rückseite 70 des Testelements 20 her erfasst wird. Zu diesem Zweck sind die Lichtquelle 32 und der Detektor 40 in dem Halbraum unterhalb der durch die Mittellinie 74 des Testbands verlaufenden Ebene angeordnet, so dass das spiegelnd reflektierte Licht im Wesentlichen in den Halbraum darüber abgestrahlt wird. Zu dieser Konfiguration durchgeführte Berechnungen zeigen, dass der Anteil des im Detektor erfassten spiegelnd reflektierten Lichts zum gesamten emittierten Licht der Lichtquelle kleiner als 0,001 ppm ist, während das Verhältnis aus detektiertem und emittiertem Licht etwa 0,13% beträgt. Somit ist also sichergestellt, dass ganz überwiegend nur diffus reflektiertes Licht aus der Reagenzschicht des Testelements 20 erfasst wird.

Zusammenfassend ist folgendes festzuhalten: Die Erfindung betrifft ein Testsystem zur Untersuchung von Körperflüssigkeiten, insbesondere Blut, mit einem eine Vielzahl von Testelementen 20 tragenden Testelementband 18, einer Bandumlenkung 22 für das Testelementband zur gezielten Applikation von Körperflüssigkeit und einer Lichtquelle 32 sowie einem Detektor 40 zur optischen Untersuchung von mit Körperflüssigkeit beaufschlagten Testelementen 20. Erfindungsgemäß wird vorgeschlagen, dass die Bandumlenkung 22 ein zum Transport des Testelementbands 18 drehbares Optikelement 24 als Umlenkrolle im Strahlengang zwischen Lichtquelle 32 und Detektor 40 aufweist.

## Patentansprüche

1. Testsystem zur Untersuchung von Körperflüssigkeiten, insbesondere Blut, mit einem eine Vielzahl von Testelementen (20) tragenden, vorzugsweise in einer Bandkassette (16) aufgewickelten Testelementband (18), einer Bandumlenkung (22) für das Testelementband (18) zur gezielten Applikation von Körperflüssigkeit und einer Lichtquelle (32) sowie einem Detektor (40) zur optischen Untersuchung von mit Körperflüssigkeit beaufschlagten Testelementen (20), **dadurch gekennzeichnet, dass** die Bandumlenkung (22) ein zum Transport des Testelementbands (18) drehbares Optikelement (24) im Strahlengang zwischen Lichtquelle (32) und Detektor (40) aufweist.

2. Testsystem nach Anspruch 1, **dadurch gekennzeichnet, dass** das lichtdurchlässige Optikelement (24) eine an das Testelementband (18) anliegende und in dessen Transportrichtung drehbare Umlenkfläche (30) aufweist.

3. Testsystem nach Anspruch 1 oder 2, **dadurch gekennzeichnet, dass** das Optikelement (24) durch eine das Licht der Lichtquelle (32) auf das jeweils zu untersuchende Testelement (20) des Testelementbands (18) bündelnde Linse gebildet ist.

4. Testsystem nach einem der Ansprüche 1 bis 3, **dadurch gekennzeichnet, dass** das Optikelement (24) als eine um ihre Längsachse (42) frei drehbar gelagerte, das Testelementband (18) mantelseitig führende zylinderförmige Linse ausgebildet ist.

5. Testsystem nach einem der Ansprüche 1 bis 4, **dadurch gekennzeichnet, dass** das Optikelement (24) einen vorzugsweise kreisförmigen, elliptischen oder vieleckigen drehsymmetrischen Querschnitt besitzt.

6. Testsystem nach einem der Ansprüche 1 bis 5, **dadurch gekennzeichnet, dass** das Optikelement (24) in einem Gleitlager, vorzugsweise einem Zapfenlager (36) oder Spitzenlager (46) drehbar gelagert ist.

7. Testsystem nach einem der Ansprüche 1 bis 6, **dadurch gekennzeichnet, dass** das Optikelement (24) in Kombination mit einer der Lichtquelle (32) nachgeordneten und/oder dem Detektor (40) vorgeordneten Linse (34) eine Abbildungsoptik bildet.

8. Testsystem nach einem der Ansprüche 1 bis 7, **dadurch gekennzeichnet, dass** zur Abschirmung von Umgebungslicht eine Blende im Bereich einer Zwischenabbildung zwischen Testelementband (18) und Detektor (40) angeordnet ist.

9. Testsystem nach einem der Ansprüche 1 bis 8, **dadurch gekennzeichnet, dass** der Detektor (40) außerhalb des Ausfallsbereichs des von dem untersuchten Testelement (20) spiegelnd reflektierten Lichts der Lichtquelle (32) angeordnet ist.

10. Testsystem zur Untersuchung von Körperflüssigkeiten, insbesondere Blut, mit einem eine Vielzahl von Testelementen (20) tragenden, vorzugsweise in einer Bandkassette (16) aufgewickelten Testelementband (18), einer Bandumlenkung (22) für das Testelementband (18) zur gezielten Applikation von Körperflüssigkeit und einer Lichtquelle (32) sowie einem Detektor (40) zur optischen Untersuchung von mit Körperflüssigkeit beaufschlagten Testelementen (20), **dadurch gekennzeichnet, dass** die Bandumlenkung (22) durch ein vorzugsweise drehbares Optikelement (24) im Strahlengang zwischen Lichtquelle (32) und Detektor (40) gebildet ist, und dass der Detektor (40) außerhalb des Ausfallsbereichs des von dem untersuchten Testelement (20) spiegelnd reflektierten Lichts der Lichtquelle (32) angeordnet ist.

11. Testsystem nach Anspruch 9 oder 10, **dadurch gekennzeichnet, dass** der Detektor (40) im Wesentlichen das an dem Testelementband (18) diffus zurückgeworfene Licht der Lichtquelle (32) erfasst.

12. Testsystem nach einem der Ansprüche 9 bis 11, **dadurch gekennzeichnet, dass** die Lichtquelle (32) unter einem Einfallswinkel und der Detektor (40) unter einem im Vergleich dazu größeren Empfangswinkel in einer Ebene einseitig von dem Einfallslot auf das Testelementband (18) ausgerichtet sind.

13. Testsystem nach einem der Ansprüche 9 bis 11, **dadurch gekennzeichnet, dass** die Lichtquelle (32) und der Detektor (40) in einem gemeinsamen Halbraum im seitlichen Abstand von einer durch die Mittellinie (74) des Testelementbands (18) im Bereich der Bandumlenkung (22) aufgespannten Begrenzungsebene angeordnet sind.

14. Testsystem nach einem der Ansprüche 1 bis 13, **dadurch gekennzeichnet, dass** die Lichtquelle (32) und der Detektor (40) auf einem Träger (60) in einem die Bandkassette (16) aufnehmenden Gerät (12) angeordnet und über eine in der Bandkassette (16) integrierte Umlenkoptik, insbesondere einen Spiegel (62) auf die Bandumlenkung (22) ausgerichtet sind.
